# EUROPEAN PATENT APPLICATION

(11) **EP 2 940 612 A1**
(43) Date of publication of application: **04.11.2015**
(21) Application number: 14166885.5
(22) Date of filing: 02.05.2014
(51) Int. Cl.: G06F 19/00

(54) **Medical assistance method and system with a medical kiosk**

(71) Applicant: Solie, Leonard, Tampa, FL 33611 (US)
(72) Inventor: Solie, Leonard, Tampa, FL 33611 (US)
(74) Representative: Romano, Giuseppe

(57) **Abstract**

A real time remote medical assistance method includes a medical kiosk for servicing a patient with a medical ailment. The kiosk provides a video feed link with a health care professional for allowing the patient and the health care professional to communicate. The patient enters personal information, self-performs biometric evaluations, displays affected body parts to the health care professional through a digital display device and a camera, and receives real time medical advice from the health care professional. Prior medical history of the patient is transmitted from an external storage device for the health care professional to review. The health care professional can then asses the medical condition of the patient. The health professional authorizes prescriptions and medicine to dispense from the kiosk. The patient makes payments and other transactions through the kiosk.

## Description

### CROSS-REFERENCE TO RELATED APPLICATION

This Non-Provisional Utility application claims the benefit of co-pending United States Provisional Patent Application Serial No. 61/606,095, filed on March 2, 2012, which is incorporated herein in its entirety.

### FIELD OF THE INVENTION

The present invention relates generally to a system and method for remote medical assistance, and more particularly to a medical kiosk with video access to a health care professional for receiving real time remote medical advice and for receiving medicine dispensed at the kiosk.

### BACKGROUND OF THE INVENTION

Medical care is a need for every individual and every family. Many families have one or more family doctors that they see for wellness visits and for the treatment of various illnesses and injuries. However, as the population of the country grows and ages, the need for doctors and health care providers in general increases, and is evidenced by a current shortage of doctors. This increased need for medical professionals and doctors in particular is not expected to abate in the near or intermediate future. In fact, the shortage is expected to worsen in the near future as the effects of new health care legislation are realized. Reports and studies indicate that within the next few years there will be a shortage of over 50,000 doctors and within the next 15 years the shortage is expected to exceed 100,000 doctors. Thus, there will be an increased reliance on alternative means of obtaining quality health care.

Medical facilities in general have been attempting to improve efficiency through automation. In particular, some aspects of patient care have been computerized such as through the use of electronic terminals interactively accessible to the patient. Some function as patient check-in stations at hospitals or doctors' offices. Other, more advanced electronic stations can perform basic diagnostic tests on patients and fall within the description of interactive kiosks. In general terms, an interactive kiosk is a computer terminal designed within a public exhibit that features specialized hardware and software for providing access to information and applications for communication, commerce, entertainment, and education. Kiosks dedicated for use in the health field can be labeled as interactive medical kiosks.

Hospitals and medical clinics recently began utilizing interactive medical kiosks to allow patients to perform routine activities. Kiosks permitting patients to check-in for their scheduled appointment and update their personal demographics reduce the need to line up and interact with a registration clerk. In areas where patients must make a co-pay, kiosks are also utilized to collect payment. As the requirements for documentation, waivers and consent increase, kiosks with integrated signature capture devices are able to present the documentation to the patient and collect their signature. These kiosks may merely appear as a computer terminal in a dedicated area of a doctor's office.

Generically, early interactive kiosks sometimes resembled telephone booths, but can also include a bench or chair for the user to sit on. Interactive kiosks are typically placed in high foot traffic settings such as hotel lobbies or airports. Integration of technology permit kiosks to perform a wide range of functions, which have evolved into self-service kiosks. For example, kiosks may enable users to enter a public utility bill account number in order to perform an online transaction, or collect cash in exchange for merchandise. Customized components such as coin hoppers, bill acceptors, card readers and thermal printers enable kiosks to meet the owner's specialized needs.

Medical kiosks can be either manned or unmanned. Manned medical kiosks can do more tests than unmanned ones. The manned medical kiosk also provides a real health care professional to assist the patient. However, the disadvantage of the manned medical kiosk is that a registered nurse has to be on site. Unmanned kiosks, on the other hand require only monthly maintenance. Furthermore, basic medical tests (blood pressure, spirometry, heart rate and ECG, blood glucose, and height/weight/BMI) can be accomplished with either manned or unmanned medical kiosks.

Even though the above cited medical kiosks address some of the needs of the market, the health care field has a need for increased automation for the care of the population in general. Specifically, there is a need for an unmanned medical kiosk that can provide medical diagnoses, treatment, prescriptions, and even medication through remote interaction with a health care professional in real time utilizing a live video feed link, while facilitating the patient to perform self-service biometric testing with full access to the patient's medical data.

### SUMMARY OF THE INVENTION

The present disclosure is generally directed to a system for providing real time remote medical diagnoses and treatment of an ailment that satisfies the need for providing facilities for remote interaction with a health care professional in real time. The system includes a medical services company for providing real time remote medical diagnoses and treatment. The company has a central office with a central computer and a memory storage unit. The central computer hosts an executable instruction set thereon for performing remote medical diagnoses and treatment. A network terminal is accessible by a health care professional for diagnosing and providing treatment of an ailment and a medical kiosk is remotely located from the network terminal of the health care professional. The medical kiosk further includes a kiosk terminal having a display device, input device and a processor. The kiosk also includes at least one biometric device for obtaining a biometric measurement of the health of a patient utilizing the medical kiosk, a storage vault for storing dispensable medications, and a medication dispenser. An electronic network is electronically communicative with the network terminal accessible by the health care professional and with the kiosk terminal.

In another aspect, the system also includes a portable personal electronic device of the type capable of two-way communications and hosting an executable application for communicative interaction with said central computer for diagnosing and treating an ailment of the user of said portable personal electronic device.

In still another aspect, the network terminal accessible by the health care professional is located remotely from the medical services company.

In yet another aspect, the medical kiosk further includes a printer communicative with the kiosk terminal for printing a prescription.

In a still further aspect, the medical kiosk further includes a payment accepting device that is capable of accepting at least one of the group of currency bills, credit cards, and insurance cards, for payment of fees relating to the diagnosis and treatment of an ailment.

In another aspect, the at least one biometric device is selected from the group consisting of a weight scale, a blood pressure monitor, a body mass index tester, a vision tester, and a thermometer.

In another aspect, the medical kiosk further includes a multi-purpose camera communicative with the kiosk terminal for capturing and communicating visual images of the patient to the network terminal accessible by the health care professional.

In a still further aspect, a medical kiosk for providing remote real time patient ailment diagnoses and treatment includes a booth defining a chamber for receiving a patient therein for permitting the patient to communicate with a remotely located health care professional. Also included is a kiosk terminal having a display device, an input device and a processor, and at least one biometric device for obtaining a biometric measurement of the health of a patient utilizing the medical kiosk. A storage vault stores dispensable medications by a medication dispenser.

In yet another aspect, the kiosk includes a printer communicative with the kiosk terminal for printing a prescription.

In another aspect, the booth provides noise attenuation to provide the patient with privacy.

In still another aspect, the kiosk also includes a payment accepting device capable of accepting at least one of the group of currency bills, credit cards, and insurance cards, for payment of fee relating to the diagnosis and treatment of an ailment.

In yet another aspect, the at least one biometric device is selected from the group consisting of a weight scale, a blood pressure monitor, a body mass index tester, a vision tester, and a thermometer.

In another aspect, the kiosk also includes a multi-purpose camera communicative with the kiosk terminal for capturing and communicating visual images of the patient to the network terminal accessible by the health care professional.

In still another aspect, the kiosk terminal is electronically communicative with an external electronic network.

In yet another aspect, a method for real time remote medical diagnoses and treatment, said method includes providing an executable instruction set hosted on a system of the type wherein the central office of a medical services company has a central computer and memory storage connected to an electronic network, a network terminal accessible by a health care professional for diagnosing and treating a patient's ailment, and a medical kiosk remotely located from the medical services company and communicative therewith via the electronic network. Medical history data and current ailment symptoms are received from a patient utilizing the medical kiosk, and intake biometric measurements are performed on the patient. The patient is interlinked with a health care professional remotely located from the medical kiosk. The ailment of the patient is diagnosed by the medical professional, and the health care professional provides care instructions to the remotely located medical kiosk for use by the patient.

In a still further aspect, the method also includes providing at least one biometric measurement device in the medical kiosk and further wherein the performance of intake biometric measurements includes the patient utilizing the biometric measurement device to obtain a biometric measurement of the patient's health.

In another aspect, the biometric measuring device is provided from a group consisting of a weight scale, a blood pressure monitor, a body mass index tester, a vision tester, and a thermometer.

In another aspect, the method includes providing a prescription for medication to the patient.

In a still further aspect, a medicine vault having at least one prescription medication stored therein is provided.

In yet another aspect, the method includes the additional step of remotely dispensing a prescription medication from the medicine vault for treatment of the patient's ailment.

These and other features, aspects, and advantages of the invention will be further understood and appreciated by those skilled in the art by reference to the following written specification, claims and appended drawings.

### BRIEF DESCRIPTION OF THE DRAWINGS

The invention will now be described, by way of example, with reference to the accompanying drawings, in which:
FIG. 1 presents a rear perspective view of a medical kiosk and a partial view of the kiosk interior;
FIG. 2 presents a side perspective view of the medical kiosk and a partial view of the kiosk interior;
FIG. 3 presents a view of the front interface panel as typically seen by a patient within the kiosk;
FIG. 4 presents a pictorial diagram of the interaction of a health care professional and a patient utilizing a medical kiosk according the present invention; and
FIG. 5 presents a schematic depiction of a network based system for providing health care utilizing a remote interactive medical kiosk; and
FIG. 6 presents a flow chart of a method for providing remote medical care utilizing a network based system of medical kiosks.
Like reference numerals refer to like parts throughout the various views of the drawings.

### DETAILED DESCRIPTION OF THE INVENTION

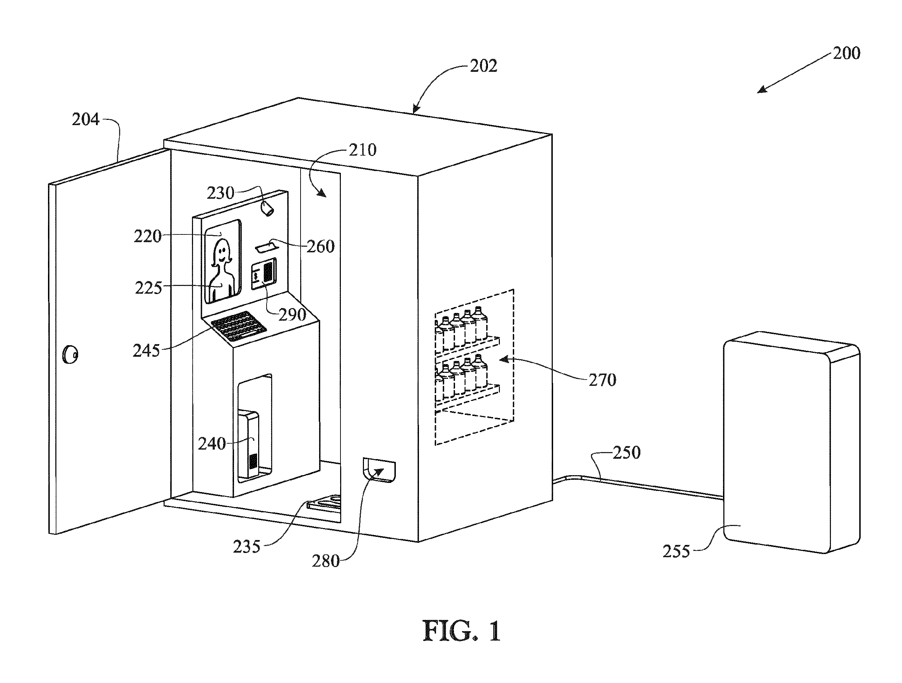

The following detailed description is merely exemplary in nature and is not intended to limit the described embodiments or the application and uses of the described embodiments. As used herein, the word "exemplary" or "illustrative" means "serving as an example, instance, or illustration." Any implementation described herein as "exemplary" or "illustrative" is not necessarily to be construed as preferred or advantageous over other implementations. All of the implementations described below are exemplary implementations provided to enable persons skilled in the art to make or use the embodiments of the disclosure and are not intended to limit the scope of the disclosure, which is defined by the claims. For purposes of description herein, the terms "upper," "lower," "left," "rear," "right," "front," "vertical," "horizontal," and derivatives thereof shall relate to the invention as oriented in FIG. 1. Furthermore, there is no intention to be bound by any expressed or implied theory presented in the preceding technical field, background, brief summary or the following detailed description. It is also to be understood that the specific devices and processes illustrated in the attached drawings, and described in the following specification, are simply exemplary embodiments of the inventive concepts defined in the appended claims. Hence, specific dimensions and other physical characteristics relating to the embodiments disclosed herein are not to be considered as limiting, unless the claims expressly state otherwise.

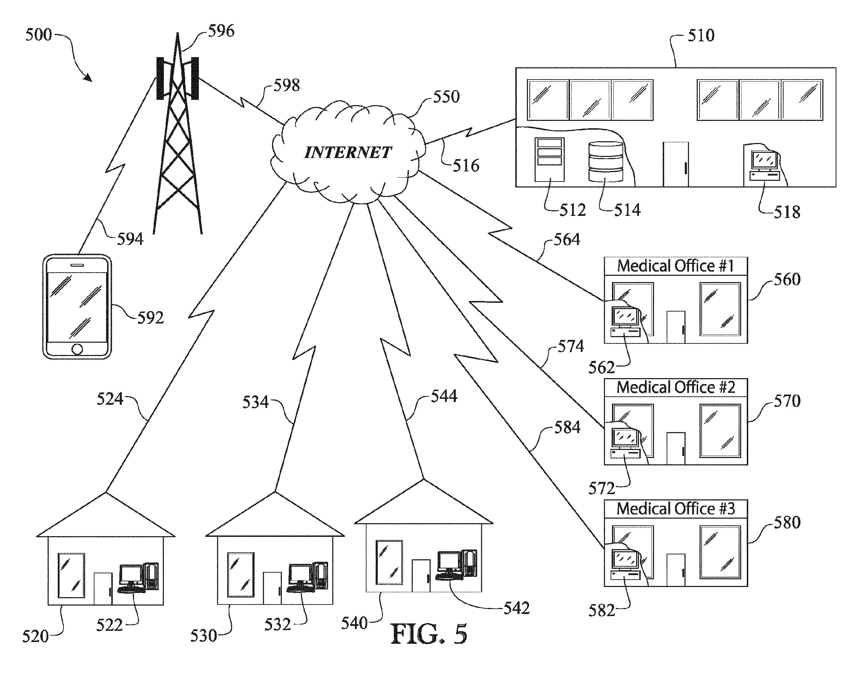

Initially referring to FIG. 5, a remote real time medical assistance system 500 is illustrated wherein a central office 510, from which the remote medical assistance host company is operated, includes a central computer 512 and a memory bank 514 communicative therewith for storing medical and patient data thereon. The central computer 512 hosts an executable instruction set for administering the remote real time medical assistance and further is communicative with an electronic network 550 such as the Internet via an electronic communication link 516. The electronic network 550 can utilize known security precautions such as encryption, passwords, limited Wi-Fi range, and the like. The central office 510 can also have therein one or more medical professionals such as nurses, physician assistants, and doctors with access to the network terminals 518 for providing remote medical consultation and assistance. The network terminals 518 are typically electronically linked to the central computer 512 for electronic communication throughout the system 500 and with entities outside of the central office 510.

One or more medical offices 560, 570, 580, participate in providing the necessary medical professionals for consultation through the central office 510. The remote real time medical assistance system 500 also includes at least one and typically a plurality of remotely located and disbursed medical kiosks 520, 530, 540. The kiosks 520, 530, 540 each have a network terminal 522, 532, 542, respectively, that is further electronically linked to the Internet 550 via the electronic communication links 524, 534, 544. Alternatively, remote medical assistance can be obtained utilizing a personal electronic device 592 such as a 'smart phone' having a central processor capable of executing downloadable application software. The personal electronic device 592 is communicative with the central office 510 utilizing the communication link 594 to communicate with the communications network 596 which is, in turn, communicative with the Internet 550 via the communications link 598.

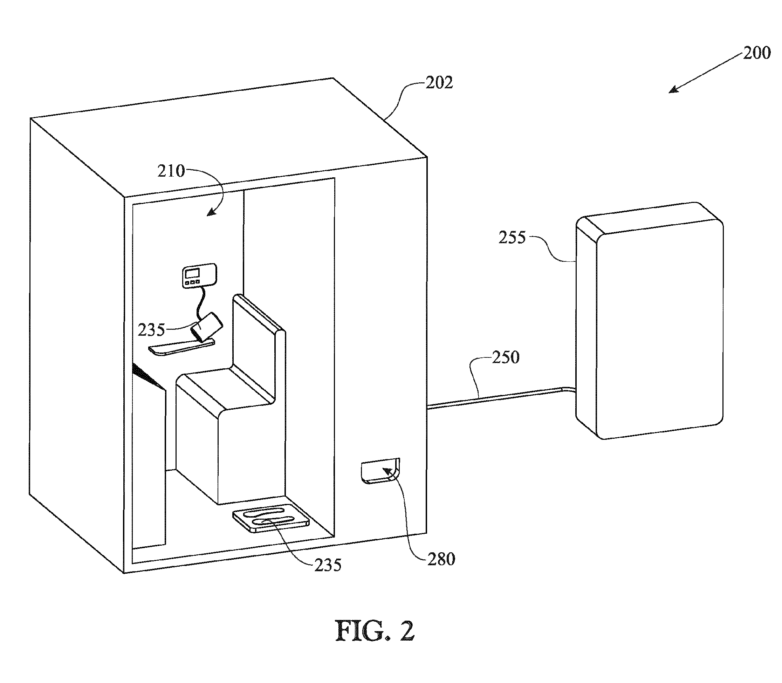

In one exemplary implementation of the invention, the medical kiosks 520, 530, 540 can be configured such as a medical kiosk 200 as shown in FIGS. 1-4 which illustrate its various components. The medical kiosk 200 includes a booth 202 that defines a chamber 210 in which a patient 300 can enter and begin obtaining remote medical assistance. A closable door 204 provides an entry location to the chamber 210. The booth 202 and door 204 can be constructed such that the booth 202 provides noise attenuation to provide the patient inside the booth 202 with maximum privacy. Such medical assistance does not include care for life-threatening medical conditions or trauma requiring the facilities and medical professionals found in an hospital emergency room, but rather is intended for such care as having an ailment diagnosed and obtaining treatment for the ailment. The kiosk 200 provides the patient 300 with easy access to a health care professional 330 twenty four hours a day, seven days a week. In one embodiment, the kiosk 200 can be located in a public shopping center such as a mall or strip shopping center, an airport, or other easily accessible public area. However, in another embodiment, the kiosk may be positioned inside a medical facility, and serve as a preexamination site prior to engaging with the medical professional in person.

Those skilled in the art, in light of the present teachings, will recognize that the patient 300 may prefer to maintain privacy relative to the medical consultation and the medical condition presented at the kiosk 200. The kiosk 200 chamber 210 provides privacy to the patient 300 by using noise barriers to reflect or absorb the energy of the sound waves, using damping structures such as sound baffles, or using active anti-noise sound generators to effectively render the chamber 210 soundproof and thereby provides this privacy function. The majority of the consultation for the real time medical assistance in kiosk 200 occurs inside the soundproof chamber 210.

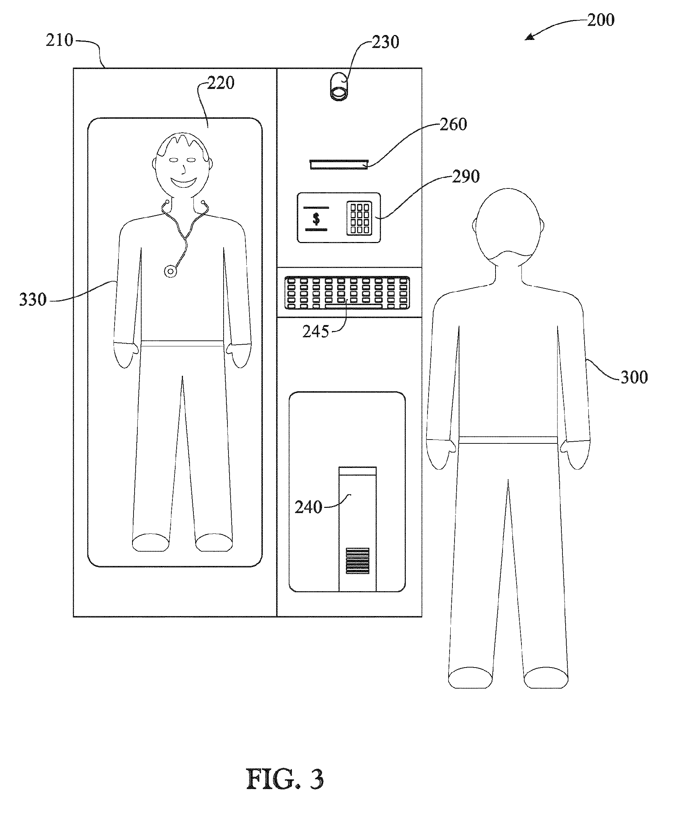

Within the medical kiosk 200, a display device 220 is available for providing a video feed link 225 between the patient 300 and the health care professional 330 over the network 550 (FIG. 5) whereby a secure, real time, two-way communication between the patient 300 and the health care professional 330 is possible. The healthcare professional 330 can be a nurse, a physician's assistant or a doctor. In one embodiment, the digital display device 220 can be a life-size screen that displays the health care professional 330 in real time and face to face to provide more realism for the patient 300. The medical kiosk 200 can also be manned with an attendant who may also be a medical professional such as a nurse. The digital display device 220 also includes audio capabilities that permits the health care professional 330 and the patient 300 to speak to one another, not shown. In FIG. 1, The digital display device 220 can be a touch screen for inputting the personal information data sample 350, accessing instructions, taking written tests, and the like. However, in other embodiments, one or more input devices 245 such as a keyboard, a mouse, or voice recognition software may integrate with the digital display device 220 FIG. 3. The display device 220 and input device 245 are interoperably controlled by a processor 240 for communicating with network 550. The display device 220, the input device 245, and the processor 240 are collectively known as the kiosk terminal.

In some embodiments, the medical kiosk 200 includes a camera 230 for permitting the medical professional 330 to view the patient 300 and any visible symptoms of the medical ailment 310 presented by the patient 300. The camera 230 allows the patient 300 to communicate with the health care professional 330 through the video feed link 225 by positioning, speaking, and displaying affected body parts in front of the camera 230. The camera 230, located in close proximity to the digital display device 220, permits the patient 300 to display a close up view of the affected body part, such as the retina, skin, or throat. In one preferred embodiment, the camera 230 captures real time video to permit the patient 300 to communicate with a health care professional 330 located at a remote medical office 560, 570, 580 (FIG. 5). The camera 230 can also be utilized to confirm the identity of the patient 300, or provide security to the kiosk 200. The camera 230 may be a multi-purpose camera capable of recording video, still shots, and zooming.

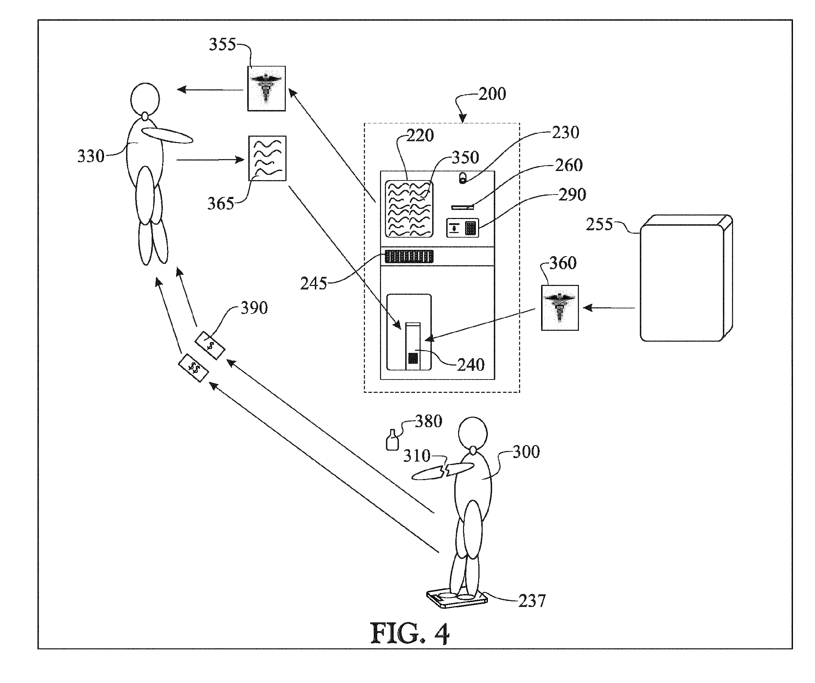

To aid in assessing the medical condition of the patient 300, the medical kiosk 200 includes one or more biometric measurement devices 235 of FIG.1, such as weight scale, blood pressure monitor, body mass index tester, vision tester, thermometer, psychological evaluation, and the like. The configuration of the biometric measurement devices 235 permit the patient 300 to perform at least one self-service biometric test 237 of FIG. 4, to create a medical data sample 355. However, in some embodiments wherein the medical kiosk 200 is attended by a medical professional, a nurse or medical technician could assist the patient 300 with the self-service biometric test 237 inside the medical kiosk 200.

In FIG. 4, the medical kiosk 200 further includes the processor 240 for storing a personal information data sample 350, storing the medical data sample 355, and receiving a prior medical data sample 360 of the patient 300 from an external storage device 255. In one preferred embodiment, the health care professional 330 is capable of accessing the external storage device 255 to view the prior medical data sample 360. Those skilled in the art will recognize that the prior medical data sample 360 permits the health care professional 330 to have a historical perspective of the medical history of the patient 300 before diagnosing the medical ailment 310.

The kiosk 200 of Fig. 4, further includes an input device 245 for inputting the personal information data sample 350 into the processor 240. In this manner, the medical condition of the patient 300 may be documented for future medical assessments and treatment. Those skilled in the art can appreciate that the input device 245 can evolve with technological advances. Suitable input devices 245 may include a keyboard, a mouse, and voice recognition software. A printer 260 for printing medicine prescriptions 365 is communicative with the processor 240 in response to communications from the health care professional 330 over the secure network 550 of FIG. 5. In this manner, the secure network 550 permits transmitting the personal information data sample 350, the medical data sample 355, and the prior medical data sample 360 between the patient 300 and the health care professional 330.

Once the health care professional 330 is able to give the patient 300 a diagnosis for the medical ailment 310, the health care professional can recommend possible treatment options, and authorize a medicine prescription 365 which can be printed on the printer 260 and made available to the patient 300. The medical kiosk 200 also can include a vault 270 for storing one or more medicines 380. The medications 380 can include common over-the-counter medications and commonly prescribed prescription medications such as, but not limited to, cough medicine, pain killers, antibiotics, skin cream, and the like. Due to the valuable nature and legal regulations of the medicines 380, the medicine vault 270 of FIG. 1, may include various security features, including titanium steel fabrication, password encryption, and an alarm system that activates upon tampering with the vault 270. The kiosk 200 also includes a medicine dispenser 280 for dispensing the medicines 380 of FIG. 4, prescribed by the medical professional 330 in lieu of printing the prescription 365 for the patient 300 to obtain at a pharmacy.

Since the remote medical assistance system 500 operates on a 'fee for service' basis, the kiosk 200 also includes a financial transaction portion 290 of FIG.3, for receiving payment in the form of cash or credit cards for the medical services provided by the health care professional 330, the medicine prescription 365, and any medicines 380 dispensed from the vault 270. However, in one alternative embodiment, the financial transaction portion 290 may be operable to accept and transact payments from insurance cards, Medicare, and various medical subsidies. The financial transaction portion 290 may include, without limitation, a credit card reader, a bill acceptor, quick response code reader, or the like.

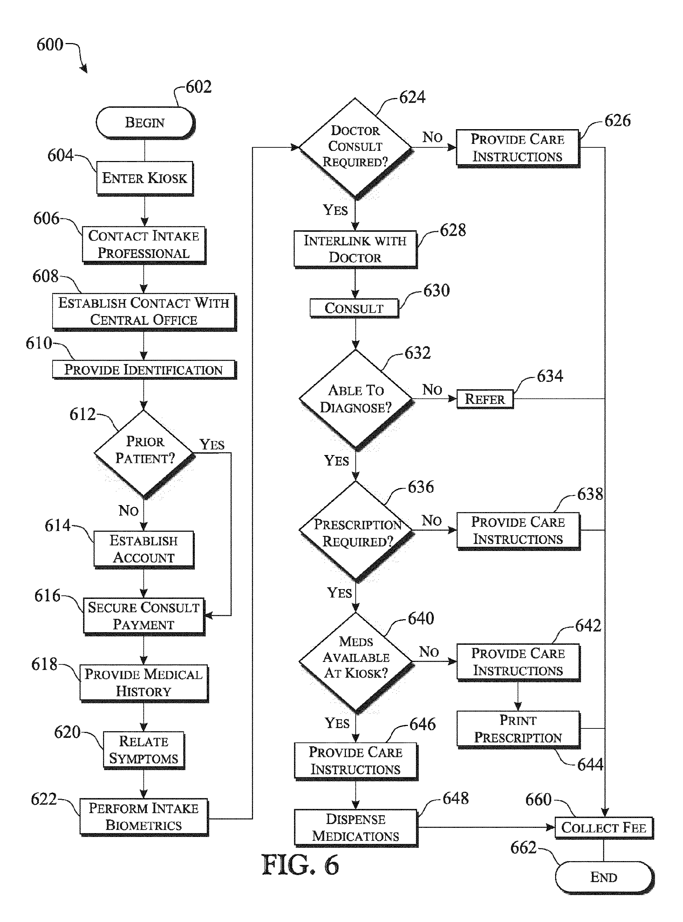

Turning now to FIG. 6, a process 600 for providing remote real time medical assistance utilizing a medical kiosk 200 as part of a remote real time medical network 500 as described above is shown illustrating its various steps. In block 602 the process starts wherein a patient 300 approaches a medical kiosk 200 that may be located at a mall, a super market, or a pharmacy. In block 604 the patient 300 enters the medical kiosk 200 and closes the door 204 to provide desired privacy during the medical consultation. Once inside the kiosk 200, the patient initiates contact with an intake professional in block 606. Contact with the intake professional can be remotely over the network 550 utilizing the electronic communication link or alternatively in person if the intake professional is manning the medical kiosk 200. In block 608 contact is established with the central office 510 utilizing the electronic communication link and the camera 230, the input device 245 and the digital display 220 in the medical kiosk 200.

Once communication with the central office 510 is established, the patient 300 provides personal identification such as name, gender, race, date of birth, allergies, etc. in block 610 using the input device 245. The patient 300 is then queried in block 612 whether the patient 300 is a prior patient. If the patient 300 is not a prior patient, then in block 614 an account for the patient 300 is established and proceeds to block 616. If the patient 300 is a prior patient and already has an account, then the process proceeds directly to block 616 where payment for the consultation is secured. Payment is made through use of currency accepting device 290 which may also function to accept credit cards or insurance cards.

In block 618 the patient can provide an existing medical history to aid the consulting medical professional in diagnosing the ailment of patient 300 by answering questions interactively with displays projected on display device 220 or by inputting an electronic file of the patient's medical history via an input device capable of receiving electronic data files. In block 620 the patient 300 relates to the intake professional or the initial health care professional currently communicating with the patient 300 the patient's symptoms and perceived ailment for which the patient 300 desires treatment. In block 622 intake biometrics are performed either by patient use of one or more biometric measurement devices 235 such as weight scale, blood pressure monitor, body mass index tester, vision test, psychological evaluation, and the like. Alternatively some or all of the biometric measurements can be taken in person by a health care professional, such as a nurse, manning the medical kiosk 200.

After receiving the patient history, biometric test results, and the patient related ailment symptoms, the intake professional determines in block 624 whether a doctor or other higher qualified health care professional needs to be consulted. If higher consultation is not required, the patient 300 is provided with care instructions in block 626, the consultation fee is collected in block 660 and the process ends at block 662. However, if the intake professional determines that a doctor consultation is required, the patient 300 is communicatively interlinked with a doctor over network 550.

The patient 300 is capable of communicating with the health care professional 330 by inputting commands through the computer input device 245. The health care professional 330 appears on the digital display device 220. The digital display device 220 can be a life-size screen that displays the health care professional 330 in real time and face to face. The patient 300 then communicates with the health care professional 330 with the video feed link 225 in real time by positioning, speaking, and displaying affected body parts in front of the camera 230. Through the camera 230, which is located in close proximity to the digital display device 220, the patient 300 is capable of displaying a close up view of the affected body part, such as the retina, skin, or throat. The health care professional 300 utilizes the personal information data sample 350, the medical data sample 355, the prior medical data sample 360, and the consultation with the patient 300 to diagnose the medical ailment 310. If the doctor is not able to diagnose the ailment or if the ailment is one which cannot be properly treated remotely, the patient 300 is referred to another doctor or medical facility equipped to provide the necessary care. The consultation fee is then collected in block 660 and the process ends at block 662.

If, on the other hand, the doctor is able to diagnose the ailment in block 632 and determines that the ailment can be effectively treated remotely, a determination is made in block 636 whether medication (over the counter or prescription) is required. If no medications are required, the patient 300 is provided with care instructions in block 638, the consultation fee is collected in block 660 and the process ends at block 662. If medications are required in block 636, a determination is made in block 640 whether the medications are available to be dispensed from the medication vault 270 of the medical kiosk 200. If the medications are not available from the medical kiosk 200, the patient 300 is provided with care instructions in block 642, a prescription is printed for the patient 300 in block 644 so that the patient can fill the prescription at a pharmacy, the consultation fee is collected in block 660 and the process ends at block 662. If the medications are available from the medication vault 270, the patient 300 is provided with care instructions in block 646, and the medications are dispensed in block 648. The consultation fee and medication fee is collected in block 660 and the process ends at block 662.

Since many modifications, variations, and changes in detail can be made to the described preferred embodiments of the invention, it is intended that all matters in the foregoing description and shown in the accompanying drawings be interpreted as illustrative and not in a limiting sense. Thus, the scope of the invention should be determined by the appended claims and their legal equivalence.

## Claims

1. A system for providing real time remote medical diagnoses and treatment of an ailment suffered by a patient, said system comprising:
a medical services company for providing real time remote medical diagnoses and treatment and having a central office having a central computer, and a memory storage unit, said central computer hosting an executable instruction set thereon for performing remote medical diagnoses and treatment;
a network terminal accessible by a health care professional for diagnosing and providing treatment of an ailment;
a medical kiosk remotely located from said network terminal of the health care professional, said medical kiosk further including:
a kiosk terminal having a display device, input device and processor;
at least one biometric device for obtaining a biometric measurement of the health of a patient utilizing said medical kiosk;
a storage vault for storing dispensable medications; and
a medication dispenser; and
an electronic network electronically communicative with said network terminal accessible by the health care professional and with said kiosk terminal.

2. The real time remote medical system according to claim 1 further including a portable personal electronic device of the type capable of two-way communications and hosting an executable application for communicative interaction with said central computer for diagnosing and treating an ailment of the user of said portable personal electronic device.

3. The real time remote medical system according to claim 1 wherein said network terminal accessible by the health care professional is located remotely from said medical services company.

4. The real time remote medical system according to claim 1 wherein said medical kiosk further includes a printer communicative with said kiosk terminal for printing a prescription.

5. The real time remote medical system according to claim 1 wherein said medical kiosk further includes a payment accepting device, said payment accepting device capable of accepting at least one of the group of currency bills, credit cards, and insurance cards, for payment of fees relating to the diagnosis and treatment of an ailment.

6. The real time remote medical system according to claim 1 wherein said at least one biometric device is selected from the group consisting of a weight scale, a blood pressure monitor, a body mass index tester, a vision tester, and a thermometer.

7. The real time remote medical system according to claim 1 wherein said medical kiosk further includes a multi-purpose camera communicative with said kiosk terminal for capturing and communicating visual images of the patient to said network terminal accessible by the health care professional.

8. A medical kiosk for providing remote real time patient ailment diagnoses and treatment, said medical kiosk comprising:
a booth defining a chamber for receiving a patient therein for permitting the patient to communicate with a remotely located health care professional;
a kiosk terminal having a display device, an input device and a processor;
at least one biometric device for obtaining a biometric measurement of the health of a patient utilizing said medical kiosk;
a storage vault for storing dispensable medications; and
a medication dispenser.

9. The medical kiosk according to claim 8 further including a printer communicative with said kiosk terminal for printing a prescription.

10. The medical kiosk according to claim 8 wherein said booth provides noise attenuation to provide the patient with privacy.

11. The medical kiosk according to claim 8 further including a payment accepting device, said payment accepting device capable of accepting at least one of the group of currency bills, credit cards, and insurance cards, for payment of fee relating to the diagnosis and treatment of an ailment.

12. The medical kiosk according to claim 8 wherein said at least one biometric device is selected from the group consisting of a weight scale, a blood pressure monitor, a body mass index tester, a vision tester, and a thermometer.

13. The medical kiosk according to claim 8 further including a multi-purpose camera communicative with said kiosk terminal for capturing and communicating visual images of the patient to said network terminal accessible by the health care professional.

14. The medical kiosk according to claim 8 wherein said kiosk terminal is electronically communicative with an external electronic network.

15. A method for real time remote medical diagnoses and treatment, said method including the steps of:
providing an executable instruction set hosted on a system of the type wherein the central office of a medical services company has a central computer and memory storage connected to an electronic network, a network terminal accessible by a health care professional for diagnosing and treating a patient's ailment, and a medical kiosk remotely located from said medical services company and communicative therewith via the electronic network;
receiving from a patient utilizing the medical kiosk medical history data and current ailment symptoms;
performing intake biometric measurements;
interlinking the patient with a health care professional remotely located from the medical kiosk;
diagnosing by the medical professional, the ailment of the patient; and
providing care instructions from the health care professional to the remotely located medical kiosk for use by the patient.

16. The method for real time remote medical diagnoses and treatment according to claim 15 further including providing at least one biometric measurement device in the medical kiosk and further wherein said performing intake biometric measurements includes the patient utilizing the at least one biometric measurement device to obtain a biometric measurement of the patient's health.

17. The method for real time remote medical diagnoses and treatment according to claim 16 wherein the providing at least one biometric measurement device includes providing at least one biometric measuring device from a group consisting of a weight scale, a blood pressure monitor, a body mass index tester, a vision tester, and a thermometer.

18. The method for real time remote medical diagnoses and treatment according to claim 15 wherein said providing care instructions includes providing a prescription for medication to the patient.

19. The method for real time remote medical diagnoses and treatment according to claim 15 further providing a medicine vault having at least one prescription medication stored therein.

20. The method for real time remote medical diagnoses and treatment according to claim 19 including after the providing health care instructions step, the step of remotely dispensing a prescription medication from the medicine vault for treatment of the patient's ailment.
